Europäisches Patentamt

European Patent Office

Office européen des brevets

(11) Publication number : **0 629 343 A2**

# (12) EUROPEAN PATENT APPLICATION

(21) Application number : **94810296.7**

(22) Date of filing : **24.05.94**

(51) Int. Cl.$^5$ : **A01H 1/02, A01H 5/12**

(30) Priority : **27.05.93 EP 93810385**

(43) Date of publication of application :
**21.12.94 Bulletin 94/51**

(84) Designated Contracting States :
**AT BE CH DE DK ES FR GB GR IE IT LI LU NL
PT SE**

(71) Applicant : **SANDOZ LTD.**
**Lichtstrasse 35**
**CH-4002 Basel (CH)**
(84) **AT BE CH DE DK ES FR GB GR IE IT LI LU NL
PT SE**

(71) Applicant : **SANDOZ-PATENT-GMBH**
**Humboldtstrasse 3**
**D-79539 Lörrach (DE)**
(84) **DE**

(71) Applicant : **SANDOZ ERFINDUNGEN
VERWALTUNGSGESELLSCHAFT M.B.H.**
**Brunner Strasse 59**
**A-1235 Vienna (AT)**
(84) **AT**

(72) Inventor : **Moreau, Bernard Marie Daniel**
**Quartier du Moutail**
**F-84260 Sarrians (FR)**
**Declaration under Rule 28(4) EPC (expert
solution)**

(54) **Fungus resistant plants.**

(57)    Cultivated lettuce plants resistant to *Bremia lactucae* and comprising in their genome a non-Dm gene
mediating *Bremia lactucae* resistance, as well as methods for producing such *Bremia latucae* resistant
lettuces.

EP 0 629 343 A2

The present invention relates to *Bremia lactucae* resistance cultivated in lettuce (i.e. Lactuca sativa) plants.

## Background

The fungus, *Bremia lactucae*, is the causative agent of the disease "Downy mildew" which affects large numbers of crop lettuces annually. Lettuce breeders have been trying for many years to breed lettuces which display resistance to this fungus and have met with only partial success with respect to the incorporation of a general *Bremia lactucae* resistance in agronomically important lettuce plants.

A feature of *Bremia lactucae* is that it is able to develop new strains over relatively short time intervals. New strains of *Bremia lactucae* are continuing to appear. Currently, there are at least sixteen (16) so-called NL races of *Bremia lactucae* known (36e Beschrijvende Rassenlijst voor Groentegewassen voor de Teelt onder Glas (1992) Eds. Aalbersberg IJ and Stolk J H, Centrum voor Plantenverdelings en Reproductieonderzoek, Wageningen) and other races of *Bremia lactucae* are identified (Bonnier F J M et al (1992) Euphytica 61: 203-211). Known commercial lettuce varieties which do display resistance to some *Bremia lactucae* strains typically comprise dominant resistant (Dm) genes in their genomes, which genes are thought to operate in a gene-for-gene system wherein the Dm gene or genes correspond with dominant avirulence genes of the pathogen (Bonnier supra). Dm is the abreviation for downy mildew; the capital letter D indicates that the gene is dominant. As long as a resistance factor has not been proved to be due to a single gene, it is characterised by an R. The Dm genes corresponding with most of the R-factors have been determined (an exception is the R18 factor). The term Dm gene as used herein comprises also such R-factors.

UPOV Guidelines for the Conduct of Tests for Distinctness, Homogeneity and Stability of Lettuce TG/13/4 (updated in 1992) and TG/13/7 (updated 26. October 1993) refer to useful Dm-genes and to tests suitable for determining the presence of Dm-genes in lettuce. The useful Dm-genes listed by UPOV are Dm 2, Dm 3, Dm 4, Dm 5/8, Dm 6, Dm 7, Dm 10, Dm 11, Dm 16 and includes also the R 18 factor. UPOV states that only these should be tested on a routine basis and that special tests may be required for the presence of Dm 1, Dm 4, Dm 15 and Dm 10.

The introduction of more than one Dm gene into the lettuce plant genome has not lead to lettuce types displaying a general and durable resistance to *Bremia lactucae*. A lettuce plant having one or more Dm genes which provide for resistance to certain strains of *Bremia lactucae* may display little or no resistance to other *B. lactucae* strains.

*Bremia lactucae* resistance (Bremia resistance) thus far in agronomically important lettuce types has displayed lack of durability over time.

For all these reasons breeders have looked for alternative sources of Bremia resistance, which are based on a genetic mechanism independent to that of Dm gene mediated resistance (such type of resistance is designated hereinafter "non-Dm gene mediated resistance").

One source of non-Dm gene mediated resistance has been recognised in a species of wild lettuce, *Lactuca saligna* for a number of years. (Gustafsson I. (1989) Euphytica 40 : 227-232 ; Netzer D. et al (1976) Hortscience 11(6):612-613). The non-Dm gene resistance is known to be effective at least in some *L. saligna* plant types against all known strains of *Bremia lactucae*, in particular against all strains controlable by the useful Dm genes and R 18 factor (see UPOV supra)

Non-Dm gene mediated Bremia resistance has also been referred to in the art as "horizontal resistance" and "immunity" to denote i.a. that it operates independently from a Dm gene mediated resistance mechanism. It follows from the above that it would be highly desirable to transfer non-Dm gene mediated resistance in commercially acceptable, i.e. cultivated lettuce plants. The successful transfer of such form of Bremia resistance to *L. sativa* has not previously been reported. Attempts at crossing *L. saligna* with *L.sativa* resulted in plants having a poor phenotype with respect to plant habit and fertility (de Vries I.M. (1990) P1. Syst. Evol. 171: 233-248).

The difficulties encountered by breeders are due to various factors, including the character of the gene conferring resistance and the difficulty to successfully transfer a non-Dm gene mediated Bremia resistance from sources of Bremia resistance different form *L. Sativa* (such as wild type lettuces eg *L. saligna*) into cultivatable and therefore agronomically (and commercially) acceptable lettuces. Indeed non-Dm gene mediated Bremia resistance such as that conferred by the non-Dm gene mediated Bremia resistance found in *L. saligna* is considered to have recessive character. Furthermore, in order to breed in a non-Dm gene mediated Bremia resistance into *L. sativa*, any plant suitable for use in a breeding program must first be made fully compatible to *L. sativa plants i.e.* agronomically and commercially acceptable important lettuce plants.

It has now been found that it is possible to succesfully transfer a non-Dm gene mediated Bremia resistance from sources different from *L. sativa* into *L.sativa* plants by selection of appropriate *Bremia lactucae* resistant plants at the F2 level. Furthermore, there appears to be a link between plants displaying non-Dm gene mediated resistance at the F2 and lack of plant vigour which necessitates the use of backcrossing selected F2 plants with commercial varieties to restore plant vigour. The F2 population segregates at least with respect to the trait of *Bremia lactucae* resistance. Selected lettuce plants from the F2 comprising a non-Dm gene mediated resistance can be crossed with agronomically important lettuce lines, the products of which can then be used for further breeding.

## Detailed Description

The present invention relates to *L. sativa* lettuce plants capable of displaying a wider resp. a more stable resistance to *Bremia lactucae*, than that resistance displayed by *L. sativa* plants containing a resistance to *Bremia lactucae* based solely on Dm gene mediated resistance.

The invention provides *L. sativa* plants capable of displaying a resistance to *Bremia lactucae* wherein the resistance to *Bremia lactucae* comprises a non-Dm gene mediated resistance (cultivated lettuce plants of the invention).

The non-Dm gene mediated resistance is preferably derived from *Lactuca saligna*. Conveniently, the cultivated lettuce plants of the invention are fully fertile.

The cultivated lettuce plants of the invention may include one or more Dm genes in their genome or they may not.

Cultivated lettuce plants, i.e. *L. sativa* plants of the invention are lettuce plants suitable for eating. Substantially all seeds of *L. sativa* plants of the invention give rise to cultivated lettuce plants displaying a non-Dm gene mediated resistance to Bremia lactucae. The phenotypical characteristics of said plants are sufficiently homogenous to be commercially acceptable and to meet the standards set by the authorities for commercialisation.

The cultivated lettuce plants of the invention may be tetraploid (2n=2x=36) or diploid (ie 2n=2x=18) in character, however it is preferably diploid. The term plant as herein comprises plant parts, cells, and seeds.

Examples of plant parts include parts suitable for consumption (heads of lettuce, its leaves) and parts suitable for plant production, for example organ tissues (eg leaf, stem, roots, shoots and the like), protoplasts, somatic embryos, anthers, stamens, petioles and cells in culture and the like. The cultivated lettuce plants of the invention may also be cultivated or propagated from plant parts using plant tissue culture techniques. Such techniques are known in the art.

Cultivated lettuce plants of the invention are capable of displaying resistance to a wider range of *Bremia lactucae* strains when subjected to challenge and/or infection with *B. lactucae* strains than lettuce plants having a Dm gene or Dm genes mediated resistance per se. Thus, non-Dm gene mediated resistance is observable under test conditions and can also be observable under normal field or greenhouse conditions.

L. sativa plants of the invention can be obtained by

i) performing an initial cross wherein the genetic material of one parent is provided by a wild type lettuce plant displaying a non-Dm gene mediated *Bremia lactucae* resistance in the cotyledon and in the true leaf, and that of the other parent is provided by a *Lactuca sativa* plant non-resistant to *Bremia lactucae*, followed by selfing to the F2 generation and selecting plants displaying resistance to *B. lactucae* strains at the cotyledon stage and the leaf stage (R escape plants); then

ii)
    a) crossing said R escape plants with *Lactuca sativa* plants displaying desirable phenotype;
    b) selfing to at least the F2;
    c) selecting R escape plants therefrom; and
    d) repeating where desired steps a) to c) till lettuce plants compatible to *Lactuca sativa* plants are obtained; and

iii)
    e) crossing R escape plants selected from compatible lettuce plants with different *Lactuca sativa* plants displaying desirable phenotype,
    f) selfing to at least the F2;
    g) selecting R escape plants therefrom; and
    h) repeating where desired steps e) to g) so as to obtain *Lactuca sativa* plants; and

iv) selfing plants obtained in iii) to at least the F4

wherein at any stage in the method where a cross is made the female parent to be crossed is prevented from self-pollination.

Generally, breeding methods for obtaining plants of the invention include identifying a suitable wild type lettuce plant which is resistant to *Bremia lactuca* at the cotyledon stage and at the leaf stage. Preferably, the wild type lettuce plant is a *Lactuca saligna* plant carrying a non-Dm gene mediated resistance to *Bremia lactucae*. This can be demonstrated for example by growing a population of wild type plants and infecting them with either a cocktail of *B. lactucae* strains, or, by subjecting the plants to individual strains of *B.lactucae* over a period of time at the cotyledon stage of development followed by reinfecting with *B. lactucae* at the leaf stage and selecting those plants which display a resistance at both stages. Resistance is proven if the leaves show no visible sporulation on the leaves. Such a procedure if used during the breeding program is referred to as an R escape test ie plants which display resistance to *B. lactucae* are the R escapes. Selected wild type lettuce plants which do display resistance at both stages of leaf development are then grown into mature plants and crossed with *L. sativa* plants which do not have resistance to *B. lactucae*. Suitable *L. sativa* plant types for the initial cross are conveniently selected from *Lactuca sativa* plants displaying desirable agronomical features. All plant types can be selected on the basis of phenotype. The progeny of the initial cross may be attained through simply allowing the crossed plants to seed, collecting the F1 seed and sowing to produce plants which are then selfed, creating the F2 generation.

In an alternative, use may be made of embryo rescue techniques wherein the product of the initial cross may be harvested from crossed plants at the embryo stage and subjected to incubations on suitable media until root and shoot formation has been established. Once sufficient root and shoot formation has been established, the resultant plantlets can then be transferred to soil and grown under suitable greenhouse conditions until a population of plants is established from which suitable plants can then be selected for further use in the breeding program. Protocols for a suitable embryo rescue method are known in the art, for example, Maisonneuve B., Agronomie (1987), 7 (5): 313-319. The F1 generation is then permitted to self to the F2 generation as before, and suitable plants selected therefrom on the basis of phenotype. Suitable F2 plants are then subjected to R escape testing as outlined herein, and R escape plants crossed with suitable *Lactuca sativa* plants carrying agronomically desirable characteristics inter alia to restore vigour, selfed to F2 where suitable plants are again selected for R escape testing. The procedure is repeated until a compatible lettuce plant to *Lactuca sativa* is obtained. Compatible plants obtained which contain a non-Dm gene mediated Bremia resistance can then be used as genitor lines in any further cultivatable lettuce breeding program. A compatible lettuce plant is one which is easy to cross with a *lactuca sativa* type and is self fertile. Since lettuce is a self-pollinating plant, it is important that a suitable genitor line containing a non-Dm gene mediated *Bremia lactucae* resistance is established. In order to establish whether or not a compatible line displays a non-Dm gene mediated resistance it is subjected to infections with *Bremia lactucae* strains at the cotyledon and first true leaf stages as described herein for the wild type lettuce plant. Thus as a preferment, there is provided a compatible lettuce plant to *L. sativa* comprising a non-Dm gene mediated *Bremia lactucae* resistance in its genome. Once compatible plants to *L. sativa* are obtained, the steps of R escape testing, selection of R escape plants for crossing with *Lactuca sativa* plants, selfing to at least the F2 are repeated until an agronomically attractive *L. sativa* type is obtained which has the non-Dm mediated gene resistance incorporated into its genome. Selected plant lines containing the non-Dm gene mediated resistance are repeatedly selfed till cultivated lettuce plants are obtained. Selfing is carried out for that purpose to at least the F4, preferably to the F5, more preferably to the F6, at which generation(s) the non-Dm gene mediated resistance is considered to be stably incorporated into the genome. Such plants are then considered suitable for the production of commercial quantities of seed capable of giving rise to *lactuca sativa* plants comprising a non-Dm gene mediated *Bremia lactucae* resistance in their genomes.

Crossing of self-pollinating plants requires that the plant to be used as the female parent is prevented from self- fertilisation. This can be done by hand emasculation of the male parts of the reproductive organs which can entail physical removal of them or emasculation can be performed via chemical means, and/or the use of water on the flowers. All such methods of emasculation are well known in the art.

The man skilled in the art will appreciate that the above outlined breeding programmes would take a number of years and that the above outlined breeding programmes are suitable for the breeding of all types of cultivatable lettuce including indoor, greenhouse, open field lettuces and the like .

There now follow examples which further illustrate the invention. It is to be understood that the examples are not to be viewed as limiting the scope of the invention in any way.

## Example 1

S 3113 and S 3114 are tested for *Bremia lactucae* resistance against *Bremia lactucae* strains NI 1 through to NI 16, and further isolated strains of Bremia fungus, given internal references TV, ITA1, and GER by application of inoculum comprising a cocktail of *Bremia lactucae* strains to the cotyledon at day six (6) after sowing and making observations on the cotyledon and first leaves at plant ages thirteen (13) days and eighteen (18) days after sowing respectively. S 3113 and S 3114 are shown to be resistant to all strains of *Bremia lactucae* tested, as shown in Table 1, whereas resistances generated via Dm genes per se are susceptible to at least one strain of *Bremia lactucae*.

In Table 1, - and (-) indicates resistance, or partial resistance respectively, to *Bremia lactucae*, and (+) indicates susceptibility to *Bremia lactucae*.

**<u>Table 1</u>**

| Important DM genes: 2 3 5/8 6 7 11 16 18 | NL Races of Bremia |  |  |  |  |  |  |  |  | S&G isolates |  |  |  |  |  |  |  |
|---|---|---|---|---|---|---|---|---|---|---|---|---|---|---|---|---|---|
|  | 1 | 2 | 3 | 4 | 5 | 6 | 7 | 9 | 10 | 11 | 12 | 13 | 14 | 15 | 16 | TV | ITA1 | GER |
| 2 - - - - - - - | + | + | - | + | - | + | + | - | + | - | - | - | + | + | + | + | + | + |
| - 3 - - - - - - | - | + | - | - | + | - | + | + | + | - | - | + | + | + | + | + | + | + |
| - - 5/8 - - - - - | - | + | + | + | - | + | - | + | + | + | + | + | + | + | + | + | + | + |
| - - - 6 - - - - | - | + | + | - | - | - | + | + | + | + | + | - | + | (-) | + | + | - | + |
| - - - - 7 - - - | - | - | + | + | + | - | + | + | + | + | + | + | - | + | + | + | + | + |
| - - - - - 11 - - | - | - | - | - | - | + | - | - | - | (-) | + | + | + | + | + | - | + | + |
| - - - - - - 16 - | - | - | - | - | - | - | - | - | - | + | + | - | - | - | + | + | - | + |
| - - - - - - - 18 | - | - | - | - | - | - | - | - | - | - | - | - | - | - | - | - | + | + |
| S 3113 | - | - | - | - | - | - | - | - | - | - | - | - | - | - | - | - | - | - |
| S 3114 | - | - | - | - | - | - | - | - | - | - | - | - | - | - | - | - | - | - |
| **Frequent Dm Gene Combinations** | 1 | 2 | 3 | 4 | 5 | 6 | 7 | 9 | 10 | 11 | 12 | 13 | 14 | 15 | 16 | TV | ITA1 | GER |
| 2 - - - 7 - - - | - | - | - | + | - | + | - | + | - | - | - | - | + | + | + | + | + | + |
| 2 - - - - 11 - - | - | - | - | - | - | + | - | - | - | - | - | - | + | + | + | - | + | + |
| - 3 - - 7 - - - | - | - | - | - | + | - | + | + | + | - | - | + | + | + | + | + | + | + |
| - 3 - - - 11 - - | - | - | - | - | - | - | - | - | - | - | - | + | + | + | + | - | + | + |
| - - 5/8 - 7 - - - | - | - | + | + | - | - | - | + | + | + | + | + | - | + | + | + | + | + |
| - - 5/8 - - 11 - - | - | - | - | - | - | + | - | - | - | - | (-) | + | + | + | + | + | + | + |
| - - - 6 - 11 - - | - | - | - | - | - | - | - | - | - | (-) | + | - | + | (-) | + | - | - | + |
| 2 3 - - 7 - - - | - | - | - | - | - | - | + | - | + | - | - | - | - | + | + | + | + | + |
| 2 3 - - - 11 - - | - | - | - | - | - | - | - | - | - | - | - | - | + | + | + | - | + | + |
| - 3 5/8 - 7 - - - | - | - | - | - | - | - | - | + | + | - | - | + | + | + | + | + | + | + |
| - 3 - 6 - 11 - - | - | - | - | - | - | - | - | - | - | - | - | - | + | (-) | + | - | + | + |
| - - 5/8 6 - 11 - - | - | - | - | - | - | - | - | - | - | - | + | - | + | (-) | + | - | - | + |
| 2 - - - - - 16 - | - | - | - | - | - | - | - | - | - | - | - | - | - | - | + | + | - | + |
| - 3 - - - - 16 - | - | - | - | - | - | - | - | - | - | - | - | - | - | - | + | + | - | + |
| - - - - - 11 16 - | - | - | - | - | - | - | - | - | - | (-) | + | - | - | - | + | - | - | + |
| - 3 - - - 11 16 - | - | - | - | - | - | - | - | - | - | (-) | - | - | - | - | + | - | - | + |
| - - - - - - - 18 | - | - | - | - | - | - | - | - | - | - | - | - | - | - | - | - | + | + |
| - - - 6 - - - 18 | - | - | - | - | - | - | - | - | - | - | - | - | - | - | - | - | - | + |
| S 3113 | - | - | - | - | - | - | - | - | - | - | - | - | - | - | - | - | - | - |
| S 3114 | - | - | - | - | - | - | - | - | - | - | - | - | - | - | - | - | - | - |

### Example 2

S 3113 and S 3114 are tested for *Bremia lactucae* resistance with individual *Bremia lactucae* strains, and a cocktail of NL 16, GER and TV *Bremia lactucae* strains and compared with the resistance of different commercial lettuce varieties containing only a Dm gene mediated resistance to *Bremia lactucae*. As is seen from Table 2, S 3113 and S 3114 are resistant to all *Bremia lactucae* strains tested whereas lettuce types containing only Dm gene mediated resistance are susceptible to some strains of *Bremia lactucae*.

**Table 2**

| Variety | Dm Gene | NL 16 | Ita 1 | Mix of TV,NL 16, and GER |
|---------|---------|-------|-------|--------------------------|
| Dandie | Dm 3 | + | + | + |
| UCDm2 | Dm 2 | + | + | + |
| Judy | Dm 3, Dm 11 | + | + | + |
| Ramona | Dm 3, Dm 11 | + | + | + |
| Safier | Dm 3 + Dm 7 + Dm 16 | + | - | + |
| Capitan | Dm 11 | + | + | + |
| Mariska | R factor 18 | - | + | + |
| S 3113 | | - | - | - |
| S 3114 | | - | - | - |

Inoculum: NL 16, Ita 1, Mixture of NL 16 + GER + TV
Infection at Cotyledon Stage : Day 0 + 6 after sowing
Observations on cotyledon : Day 0 + 13 after sowing
Observations on leaves : Day 0 + 18 after sowing
- Resistant
+ Susceptible

S 3113 and S 3114 have been deposited with the National Collections of Industrial and Marine Bacteria Ltd, International Depository Authority, Scotland, on 19th May 1993 and provided with designation numbers NCIMB 40556 and NCIMB 40557 respectively.

### DESCRIPTION OF BREEDING SCHEMES

**Breeding Scheme 1:** shows an example of a breeding programme initiated to introduce a non-Dm gene mediated *Bremia lactucae* resistance into *L. sativa*. The resulting plants, S 3113, may be subjected to further self pollination. S3113 contains both the non-Dm gene mediated Bremia resistance gene and Dm genes, Dm 3 and Dm 11, in the genome .

Female plants produced in the breeding programme are on the lefthand side and male plants on the righthand side of the diagram. *Lactuca saligna* has been labelled as "B28", an inhouse designation, intimating that this plant contains an identified general non-Dm gene mediated resistance to *Bremia lactucae*. B28-60 is an inhouse designation for the first agronomically important lettuce line in which the non-Dm gene mediated Bremia lactucae resistance is incorporated and which is compatible with (ie self-compatible, fully fertile, and easily crossed with *Lactuca sativa*) *Lactuca sativa*, and is able to be used in all subsequent breeding programmes as a genitor line. B28-60 is the F3 selfing from the cross F2 x Palmyran. The name "Novir" relates to a lettuce first available from INRA which is not resistant to any strain of *Bremia lactucae*. Other names, "Palmyran", "Jessy", "Ramona", "Judy", "Thirana" and "Penny" relate to commercial varieties of S&G Seeds BV which contain Dm genes, Dm 3 and Dm 11 in their genome. "E-207", "E-99" relate to inhouse generated S&G Seeds experimental lines. Ramsal-19 is an inhouse designation for a further agronomically important lettuce line which can be grown in short day conditions and contains the B28 resistance. Ramsal-19 is used as a genitor for all greenhouse lettuce types and is the F3 selfing from the cross F2 x Ramona. The varieties listed above are all crossed with plants selected from at least the F2 which contain a non-Dm gene mediated resistance to *Bremia lactucae*. All F2 plants used for crossing and/or selfing throughout the breeding scheme are all selected R escape plants. The crosses F2 x Thirana, and F4 x Judy, are made to introduce an improved commercial phenotype with respect to commercial attractiveness, which commercial phenotype also contains by virtue of the earlier introduced B28 resistance a stably incorporated B28 resistance. Selfing to F6, ie to S 3113, is viewed as the point where B28 resistance is regarded as being stably incorporated in the genome.

**Breeding Scheme 2:** essentially follows the breeding selection pattern of Breeding Scheme 1 except that at F2 x E-207, the resulting selected (selfed) F2 plant from the cross, is crossed with a different type, "E-99", also an S&G Seeds B.V. experimental variety, and the resulting F4 derived from the F2 R escape selfing is crossed with a further commercial type, "Penny", an S&G Seeds B.V. commercial type, and selfed to the F6 from selected F2 R escape plants, giving S 3114.

## Breeding Scheme 1

### Genealogy Material L. Saligna x L. Sativa

```
Lactuca Saligna      x    Lactuca Sativa
   "B-28                      "Novir"
                               |
                               ↓
                   F2      x      "Palmyran"
               R escape Test          |
                                      ↓
F3   [ B28-60 ]  ◄———  F2     R escape  Test
                               |
                               ↓
                         F3      x    "Jessy"
                      (B28-60)          |
                                        ↓
                               F2    x   "Ramona"
                                            |
                                            ↓
F3   [ Ramsal-19 ]  ◄———  F2   x   "E-207"              o
                                       |
                                       ↓
                              F2    x   "Thirana"
                                           |
                                           ↓
                                  F4    x   Judy
                                              |
                                              ↓
                                            F6
                                          "S 3113"
```

## Breeding Scheme 2

### Genealogy Material : L. Saligna x L. Sativa

Lactuca saligna      x      Lactuca sativa
"B-28                              "Novir"

F2          x          "Palmyran"
R escape Test

F3    | B28-60 |    ◄————    F2      R escape   Test

F3          x      "Jessy"
(B28-60)

F2      x      "Ramona"

F3      | Ramsal-19 |    ◄————    F2      x      "E-207"

F2      x      "E-99"

F4      x      "Penny"

F6
"S 3114"

## Claims

1. Cultivated lettuce plants resistant to *Bremia lactucae* characterized in that they comprise in their genome a non-Dm gene mediating Bremia lactucae resistance.

2. Cultivated lettuce plants according to Claim 1 characterized in that the non-Dm gene confers resistance to the dutch Bremia races NL 1 to 7, NL 9 to 16, TV and to ITA1.

3. Cultivated lettuce plants according to Claim 1 or Claim 2 wherein the non-Dm gene is obtained from *Lac-*

9

*tuca saligna.*

4.  Cultivated lettuce plants according to any one of Claims 1 to 3 wherein the cultivatable lettuce plants are fully fertile.

5.  Cultivated lettuce plants according to any one of Claims 1 to 4 comprising in their genome a non-Dm gene and one or more Dm genes.

6.  A method of obtaining a cultivated lettuce plants according to Claims 1 to 5 which method comprises:
    i) performing an initial cross wherein the genetic material of one parent is provided by a wild type lettuce plant displaying a non-Dm gene mediated *Bremia lactucae* resistance in the cotyledon and in the true leaf, and that of the other parent is provided by a *Lactuca sativa* plant non-resistant to *Bremia lactucae*, followed by selfing to the F2 generation and selecting plants displaying resistance to B. lactucae strains at the cotyledon stage and the leaf stage (R escape plants); then
    ii)
    　　a) crossing said R escape plants with *Lactuca sativa* plants displaying desirable phenotype;
    　　b) selfing to at least the F2;
    　　c) selecting R escape plants therefrom; and
    　　d) repeating where desired steps a) to c) till lettuce plants compatible to *lactuca sativa* plants are obtained; and
    iii)
    　　e) crossing R escape plants selected from compatible lettuce plants with different *Lactuca sativa* plants displaying desirable phenotype;
    　　f) selfing to at least the F2;
    　　g) selecting R escape plants therefrom; and
    　　h) repeating where desired steps e) to g) so as to obtain *Lactuca sativa* plants; and
    iv) selfing plants obtained in iii) to at least the F4
    wherein at any stage in the method where a cross is made the female parent to be crossed is prevented from self-pollination.

7.  A method according to Claim 8 wherein the male parent of the initial cross is a *Lactuca sativa* plant and the female parent is a *Lactuca saligna* plant.

8.  A method according to Claim 8 or Claim 9 wherein the male parent used in crossing is a *Lactuca sativa* plant and the female parent carries the non-Dm gene mediated resistance to *Bremia lactucae* from *Lactuca saligna*.

9.  Cultivated lettuce plants obtainable according to the method of any one of claims 6 to 8.

10. Head or leaves of cultivated lettuce plants of any one of Claims 1 to 5 and 9.

11. Seeds or other propagation material of cultivated lettuce plants of any one of Claims 1 to 5 and 9.